# EUROPÄISCHE PATENTSCHRIFT

(11) **EP 1 278 463 B1**
(45) Veröffentlichungstag und Bekanntmachung des Hinweises auf die Patenterteilung: **10.11.2004**
(21) Anmeldenummer: 01940330.2
(22) Anmeldetag: 14.04.2001
(51) Int. Cl.: A61B 17/04, A61F 2/08

(54) **FADEN-ANKER-SYSTEM ZUM VERBINDEN VON GEWEBETEILEN**
THREAD ANCHOR SYSTEM FOR ASSEMBLING PARTS OF TISSUES
SYSTEME D'ANCRAGE DE FIL DESTINE A ASSEMBLER DES PARTIES DE TISSU

(30) Priorität: 29.04.2000 DE 10021122
(43) Veröffentlichungstag der Anmeldung: 29.01.2003
(73) Patentinhaber: Aesculap AG & Co. KG, 78532 Tuttlingen (DE)
(72) Erfinder: EICHHORN, Jürgen, 94360 Mitterfels/Scheibelsgrub (DE); GIORDANO, Nicola, 78056 Villingen-Schwenningen (DE); KIENZLE, Karl-Ernst, 78532 Tuttlingen (DE); KIRMSE, Gerhard, Bethlehem, PA 18017 (US)
(74) Vertreter: HOEGER, STELLRECHT & PARTNER Patentanwälte
(86) Internationale Anmeldenummer: PCT/EP2001/004279
(87) Internationale Veröffentlichungsnummer: WO 2001/082802

(56) Entgegenhaltungen:
- EP-A- 0 982 003
- US-A- 5 059 206
- US-A- 5 702 462
- US-A- 5 730 744
- US-A- 5 891 168
- US-A- 6 007 566

## Beschreibung

Die Erfindung betrifft ein Faden-Anker-System zum Verbinden von Gewebeteilen, insbesondere zur Versorgung von Meniskusrissen, mit mindestens zwei pfeil- oder stiftförmigen Ankerimplantaten, die über einen flexiblen, an den Ankerimplantaten festgelegten Faden miteinander verbunden sind.

Zur Verbindung von Gewebeteilen, beispielsweise den durch einen Riß voneinander getrennten Teilen eines Meniskus, ist es bekannt, in die Gewebeteile pfeilartige Ankerimplantate einzustoßen, die in den Gewebeteilen durch Widerhaken oder entsprechende widerhakenförmige Formgebung gegen ein Herausziehen gesichert sind. Diese Implantate können entweder die zu verbindenden Gewebeteile direkt verbinden, indem sie beide Gewebeteile durchsetzen (WO 85/03857; US 5,843,084), oder aber zwei derartige, pfeilförmige Ankerimplantate werden über ein flexibles Zwischenglied miteinander verbunden, so daß sie dadurch die Verbindungsstelle überbrücken (EP 0 589 306 A2; EP 0 664 198 B1).

In der US-A-5,702,462 ist weiterhin ein Faden-Anker-System beschrieben, bei dem pfeilförmige Ankerimplantate jeweils mit einem fest mit ihnen verbundenen Faden versehen sind, die Fäden verschiedener Ankerimplantate werden nach dem Einsetzen der Ankerimplantate in das Gewebe miteinander verknotet, so daß dadurch über die Fadenbrücken die zu verbindenden Gewebeteile verbunden werden. Allerdings ist dieses Verfahren relativ kompliziert, da die Fadenenden von jeweils zwei Ankerimplantaten miteinander verknotet werden müssen, außerdem bilden die Knoten eine Verdickung im Faden, die zu Gewebereizungen Anlaß geben kann.

Es ist Aufgabe der Erfindung, ein gattungsgemäßes Faden-Anker-System so auszubilden, daß es in besonders einfacher Weise angelegt werden kann und daß insbesondere die Bildung von Knoten vermieden werden kann.

Diese Aufgabe wird bei einem Faden-Anker-System der eingangs beschriebenen Art erfindungsgemäß dadurch gelöst, daß mindestens eines der Ankerimplantate einen durchgehenden Längskanal zur Aufnahme eines längsverschieblichen Kernes aufweist, daß an diesem Ankerimplantat eine Fadenführung zur längsverschieblichen Aufnahme eines allen Ankerimplantaten gemeinsamen Fadens angeordnet ist, daß der Fadenführung eine Klemmeinrichtung zur Festlegung des Fadens in der Fadenführung zugeordnet ist und daß die Klemmeinrichtung unwirksam ist, solange sich der Kern in dem Längskanal befindet, und erst wirksam wird, wenn der Kern aus dem Längskanal herausgezogen wird.

Im Gegensatz zu bekannten Ankerimplantaten werden also bei dem beschriebenen Faden-Anker-System mindestens zwei Ankerimplantate über einen gemeinsamen Faden verbunden, und an mindestens einem dieser Ankerimplantate ist dieser Faden in einer Fadenführung zunächst frei verschiebbar. Nach dem Einsetzen der Ankerimplantate in das Gewebe kann somit der Faden zwischen den beiden Ankerimplantaten in der gewünschten Weise gespannt werden, und in diesem gespannten Zustand läßt sich der Faden dann an den Ankerimplantaten dadurch festlegen, daß der Kern aus dem Längskanal herausgezogen wird. Eine Knotenbildung ist nicht mehr notwendig, und trotzdem ist es möglich, den die eingesetzten Ankerimplantate verbindenden Faden in der gewünschten Weise zu spannen und dadurch die zu verbindenden Gewebeteile gegeneinander zu fixieren.

Bei einer ersten bevorzugten Ausführungsform ist vorgesehen, daß ein erstes Ankerimplantat fest mit dem Faden verbunden ist und daß alle weiteren Ankerimplantate einen Längskanal mit herausziehbarem Kern und eine Fadenführung mit Klemmeinrichtung aufweisen. Auf diese Weise kann ausgehend von einem ersten Ankerimplantat mit festem Faden auf demselben Faden eine beliebige Anzahl weiterer Ankerimplantate zunächst verschiebbar aufgefädelt sein, nach dem Einsetzen der Ankerimplantate kann jedoch der Faden zwischen diesen Ankerimplantaten gespannt und festgelegt werden. Dabei kann das Faden-Anker-System nur zwei Ankerimplantate oder eine größere Anzahl von Ankerimplantaten umfassen.

Auch das erste Ankerimplantat kann einen durchgehenden Längskanal aufweisen, der in noch näher erläuterter Weise das Einsetzen des Ankerimplantates in das Gewebeteil erleichtert.

Bei einer anderen Ausführungsform ist vorgesehen, daß alle Ankerimplantate einen Längskanal mit herausziehbarem Kern und eine Fadenführung mit Klemmeinrichtung aufweisen, d.h. alle Ankerimplantate sind auf dem Faden zunächst frei verschiebbar und werden erst durch das Herausziehen des Kerns längs des Fadens festgelegt.

Insbesondere bei einer solchen Ausgestaltung ist es vorteilhaft, wenn das eine Ende des Fadens ein Anschlagelement trägt, welches beim Spannen des Fadens an einem Gewebeteil anliegt und dadurch das weitere Einziehen des Fadens in das Gewebeteil verhindert.

Der Kern kann ein stößelförmiges Teil sein, das aus dem Ankerimplantat zum Aktivieren der Klemmeinrichtung herausgezogen wird, es ist aber besonders vorteilhaft, wenn der Kern ein Führungsdraht ist, der durch das Ankerimplantat hindurchgeschoben ist und in das Gewebe eingestochen wird, es ist nämlich dann möglich, das Ankerimplantat längs dieses Führungsdrahtes in die gewünschte Position im Gewebe vorzuschieben. Der Führungsdraht übernimmt also bei dieser Ausgestaltung sowohl die Führung des Ankerimplantats beim Einsetzen als auch die Aktivierung der Klemmeinrichtung für den Faden beim Herausziehen des Führungsdrahtes.

Der Führungsdraht kann auch dazu dienen, die miteinander zu verbindenden Gewebeteile relativ zueinander zu positionieren, so daß beim Einführen des Ankerimplantates die Gewebeteile in der gewünschten Relativposition zueinander fixiert werden. Es kann damit ausgeschlossen werden, daß diese Gewebeteile schief oder überlappend zusammengefügt werden.

Günstig ist es, wenn die Ankerimplantate an ihrer Mantelfläche Widerhaken oder widerhakenförmige Vorsprünge tragen, so daß einmal eingesetzte Ankerimplantate in ihrer Position festgelegt werden.

Vorzugsweise bestehen das Ankerimplantat und/oder der Faden aus resorbierbarem Kunststoffmaterial, so daß diese Implantate nach Beendigung des Heilungsprozesses im Körper aufgelöst werden.

Der Kern kann die Klemmeinrichtung in unterschiedlicher Weise wirksam machen. Beispielsweise kann der Kern im eingeschobenen Zustand ein beweglich am Ankerimplantat angeordnetes Klemmelement, beispielsweise eine flexible Klemmzunge, in eine Stellung verschieben, in der dieses Klemmelement den Faden nicht wirksam blockieren kann. Sobald der Kern aus dem Ankerimplantat herausgezogen wird, legt sich dieses Klemmelement, welches beispielsweise ein Widerhaken sein kann, an den Faden und verhindert auf diese Weise eine Verschiebung des Fadens relativ zum Ankerimplantat. Es kann sich bei diesen Klemmelementen um einseitig wirkende Widerhaken handeln, aber auch um beidseitig wirkende, d.h. es werden Widerhaken für beide Verschieberichtungen des Fadens vorgesehen, so daß der Faden nach dem Herausziehen des Kerns gegen Verschiebung in beiden Richtungen festgelegt ist.

Bei einer besonders bevorzugten Ausführungsform ist vorgesehen, daß die Fadenführung einen den Längskanal durchsetzenden Klemmabschnitt und einen neben dem Längskanal angeordneten Verschiebeabschnitt aufweist, daß der in das Ankerimplantat eingeschobene Kern den Klemmabschnitt gegenüber dem Verschiebeabschnitt absperrt und daß die Klemmeinrichtung in dem Klemmabschnitt angeordnet ist, während der Faden in dem Verschiebeabschnitt frei verschiebbar ist.

Durch Herausziehen des Kerns wird bei dieser Konstruktion der Klemmabschnitt freigegeben, der Faden kann aus dem Verschiebeabschnitt in den Klemmabschnitt eintreten und wird dadurch gegen eine Längsverschiebung im Ankerimplantat festgelegt.

Die Klemmeinrichtung kann eine Vielzahl von Vorsprüngen oder Widerhaken aufweisen, die an dem Faden zur Anlage kommen, wenn der Faden in die Klemmeinrichtung gelangt, und die dadurch den Faden im Ankerimplantat festlegen.

Bei einer besonders bevorzugten Ausführungsform ist vorgesehen, daß die Klemmeinrichtung ein sich zu seinem Boden hin verengender Schlitz ist, in den der Faden eingelegt ist und zwischen dessen Seitenwänden er eingeklemmt wird, wenn er gegen den Boden des Schlitzes verschoben wird.

Dabei ist es vorteilhaft, wenn sich an den Schlitz beidseitig zum Boden des Schlitzes hin abgewinkelte Führungen für den Faden anschließen. Sobald der Faden in diesen abgewinkelten Führungen gespannt wird, wird er zwangsläufig in den Schlitz hineingezogen und gegen dessen Boden gespannt, so daß er in dem Schlitz eingeklemmt wird.

Diese sich an den Schlitz anschließenden Führungen können als seitlich offene oder geschlossene Kanäle ausgebildet sein, die zu beiden Seiten des Längskanals im wesentlichen parallel zu diesem im Ankerimplantat verlaufen und an einem Ende in den Boden des Schlitzes einmünden. Auf diese Weise treten beide Enden desselben Fadens an derselben Seite des Ankerimplantates aus diesem aus, im Inneren des Ankerimplantates bildet der Faden dabei eine U-förmige Schleife aus, die in dem Schlitz geführt wird.

Dabei ist es vorteilhaft, wenn der Schlitz zumindest im Bereich des Klemmabschnitts das Ankerimplantat diametral durchsetzt.

Die Seitenwände des Schlitzes können zum Boden hin spitzwinkelig zusammenlaufen, günstig ist es, wenn die Seitenwände im Querschnitt konkav gebogen sind.

Bei einer weiteren bevorzugten Ausführungsform ist vorgesehen, daß der durch den Schlitz gebildete Klemmabschnitt an seinem dem Boden gegenüberliegenden Ende in den außerhalb des Längskanals angeordneten, ebenfalls Teil des Schlitzes bildenden Verschiebeabschnitt übergeht, wobei die Seitenwände des Schlitzes im Verschiebeabschnitt einen Abstand voneinander haben, der größer ist als der Durchmesser des Fadens.

Die Ebene des Schlitzes kann im Verschiebeabschnitt gegenüber der Ebene des Schlitzes im Klemmabschnitt abgewinkelt sein, wobei die Abwinkelung vorzugsweise zwischen 30 und 90° liegt. Dabei ist es günstig, wenn der Schlitz im Übergangsbereich zwischen Klemmabschnitt und Verschiebeabschnitt gebogen ist, so daß der Faden nach dem Herausziehen des Kerns und beim Spannen ohne weiteres vom Verschiebeabschnitt in den Klemmabschnitt gleitet.

Es kann vorgesehen sein, daß der Schlitz am äußeren Ende des Verschiebeabschnitts an der Umfangsfläche des Ankerimplantats nach außen hin offen ist. Dies erleichtert das Einlegen des Fadens in das Ankerimplantat.

Zum Einsetzen des Faden-Anker-Systems kann ein Instrument verwendet werden, welches gekennzeichnet ist durch ein Führungsrohr zur Aufnahme eines Ankerimplantates, durch einen durch die Längsbohrung des Ankerimplantates hindurchtretenden, mit seiner Spitze nach vorn aus dem Führungsrohr herausragenden Führungsdraht und durch ein im Führungsrohr längsverschiebliches Ausschiebelement zum Herausschieben des Ankerimplantats aus dem Führungsrohr.

Mit einem solchen Instrument kann das Ankerimplantat in einfacher Weise an der gewünschten Stelle plaziert werden, indem zunächst der aus dem Führungsrohr hervorstehende Führungsdraht an die gewünschte Stelle des Gewebes vorgeschoben wird, dann wird durch Bewegung des Ausschiebelementes im Führungsrohr das Ankerimplantat längs des Führungsdrahtes aus dem Führungsrohr ausgeschoben, bis das Ankerimplantat die gewünschte Position im Gewebe erreicht.

Das Instrument bleibt angelegt, während der Faden in der gewünschten Weise gespannt wird, zur Festlegung des Fadens genügt es dann, das Instrument aus dem Gewebe herauszuziehen, dabei gibt auch der Führungsdraht den Längskanal in dem Ankerimplantat frei und aktiviert dadurch die Klemmeinrichtung im Ankerimplantat, d.h. der Faden wird dadurch festgelegt.

Vorzugsweise sind der Führungsdraht und/oder das Ausschiebeelement flexibel.

Das Ausschiebelement kann den Führungsdraht umgeben.

Es ist günstig, wenn der Führungsdraht gegenüber dem Führungsrohr längsverschieblich ist, so daß die Eintauchtiefe des Führungsdrahtes in das Gewebe einstellbar ist, d.h. der Überstand des Führungsdrahtes über das Führungsrohr.

Die nachfolgende Beschreibung bevorzugter Ausführungsformen der Erfindung dient im Zusammenhang mit der Zeichnung der näheren Erläuterung. Es zeigen:
- Figur 1:: eine Längsschnittansicht des vorderen Teils eines Instruments zum Einsetzen eines Ankerimplantates mit einem eingelegten Ankerimplantat vor dem Einschieben des Ankerimplantates in das Gewebe;
- Figur 2:: eine Schnittansicht durch zwei miteinander zu verbindende Teile eines Gewebes mit darin eingesetzten Ankerimplantaten vor dem Spannen des die Ankerimplantate verbindenden Fadens;
- Figur 3:: eine Ansicht des Gewebeteils der Figur 2 in Richtung des Pfeiles A in Figur 2;
- Figur 4:: eine Ansicht ähnlich Figur 2 nach dem Festlegen des die Ankerimplantate verbindenden Fadens an einem Ankerimplantat;
- Figur 5:: eine Schnittansicht längs Linie 5-5 in Figur 4 und
- Figur 6:: eine Ansicht ähnlich Figur 2 nach dem Festlegen des die Ankerimplantate verbindenden Fadens in beiden Ankerimplantaten.

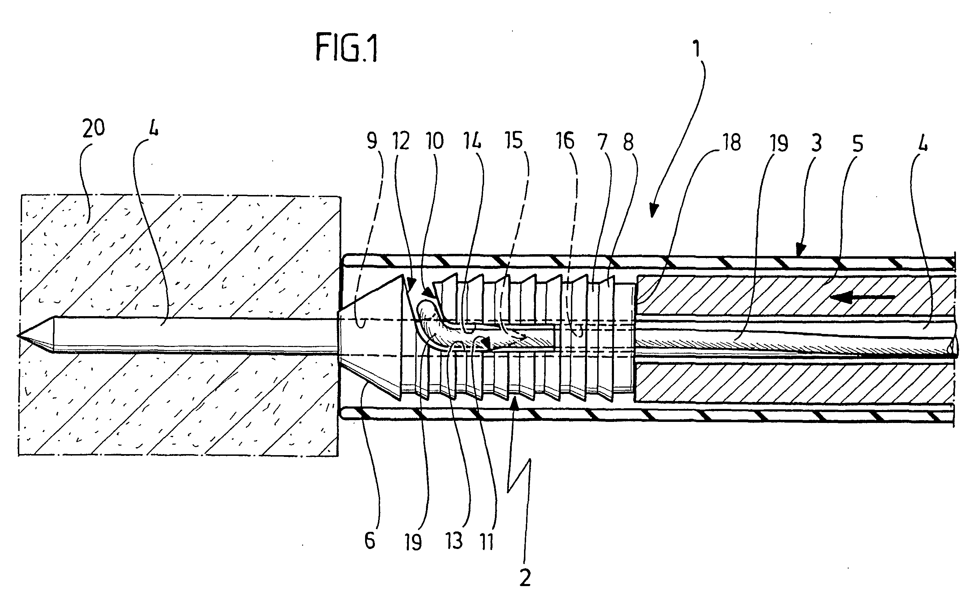

In Figur 1 ist ein Instrument 1 zum Einsetzen eines kreiszylindrischen Ankerimplantates 2 dargestellt, welches im wesentlichen ein flexibles Führungsrohr 3, einen darin zentral angeordneten, ebenfalls flexiblen und über das Ende des Führungsrohres 3 hervorstehenden Führungsdraht 4 sowie ein im Inneren des Führungsrohres 3 den Führungsdraht 4 umgebend längsverschieblich gelagertes Ausschiebelement 5 umfaßt. Der Führungsdraht 4 kann ebenfalls im Führungsrohr 3 längsverschieblich gelagert sein, die Verschiebung des Ausschiebelementes und gegebenenfalls des Führungsdrahtes erfolgt über in der Zeichnung nicht dargestellte Handhabungsgriffe am gegenüberliegenden Ende des Führungsrohres 3. Der Außendurchmesser des Führungsrohres 3 ist dabei so gewählt, daß dieses auch ohne weiteres über herkömmliche Trokarhülsen in das Körperinnere eingeführt werden kann, beispielsweise kann der Außendurchmesser des Führungsrohres 3 bei 7 bis 10 mm liegen, vorzugsweise bei 3 bis 5 mm.

Das Führungsrohr des Einsatzinstrumentes kann im übrigen direkt durch eine Hautinzision in ein Gelenk eingeführt werden, in dem ein Ankerimplantat 2 eingesetzt werden soll, beispielsweise im Meniskus. Bei anderen Indikationen wird das Führungsrohr 3 dagegen in der beschriebenen Weise durch eine Trokarhülse in den Körper eingeführt.

Das Ankerimplantat 2 ist ein im wesentlichen kreiszylindrischer, stift- oder pfeilförmiger Körper aus einem resorbierbaren Kunststoff, vorzugsweise mit einem Durchmesser von 2 bis 3 mm, der eine konische Spitze 6 und an seinem zylindrischen Außenmantel 7 eine Vielzahl von widerhakenförmigen Umfangsrippen 8 aufweist. Ein zentraler Längskanal 9 durchsetzt das Ankerimplantat 2, und dieser Längskanal 9 nimmt den Führungsdraht 4 auf, d.h. das Ankerimplantat 2 ist vom offenen Ende des Führungsrohres 3 her über den Führungsdraht 4 in den Innenraum des Führungsrohres 3 eingeschoben, so daß das Ankerimplantat 2 im Führungsrohr 3 bei zurückgeschobenem Ausschiebelement 5 vollständig aufgenommen ist, wie dies in Figur 1 dargestellt ist.

In seinem mittleren Bereich weist das Ankerimplantat 2 einen dieses quer durchsetzenden Schlitz 10 auf, der in einem Klemmabschnitt 11 parallel zur Längsrichtung des Ankerimplantates 2 dieses diametral durchsetzt und der sich an seinem der Spitze 6 zugewandten Ende in einen schräg nach außen zum Außenmantel 7 gerichteten, nach außen hin offenen Verschiebeabschnitt 12 fortsetzt. Der Verschiebeabschnitt 12 ist gegenüber dem Klemmabschnitt 11 um einen Winkel zwischen 30° und 90° geneigt, im Übergangsbereich zwischen Klemmabschnitt 11 und Verschiebeabschnitt 12 ist der Schlitz 10 gebogen ausgebildet, d.h. der Übergang erfolgt nicht stufig, sondern stetig.

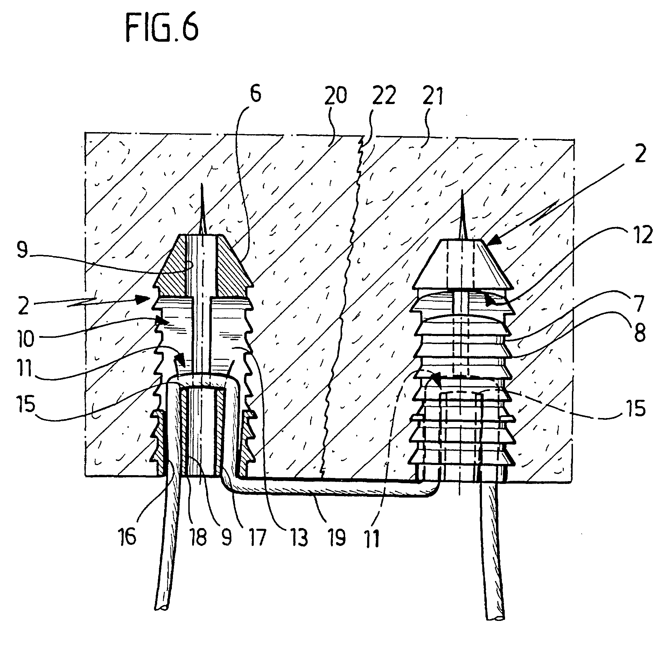

Die Seitenwände 13 und 14 des Schlitzes 10 konvergieren am Boden 15 des Schlitzes 10 und laufen dort spitzwinklig zusammen, in den daran anschließenden Wandbereichen sind die Seitenwände 13 und 14 geringfügig konkav gebogen (Figur 5).

Auf gegenüberliegenden Seiten des Längskanales 9 verlaufen parallel zu diesem zwei geschlossene Führungskanäle 16, 17 die in den Boden 15 des Schlitzes 10 einmünden und die mit ihrem anderen Ende aus der der Spitze 6 des Ankerimplantates 2 gegenüberliegenden Rückseite 18 austreten.

In das Ankerimplantat 2 ist ein flexibler Faden 19 eingelegt, der durch den einen Führungskanal 16 in den Schlitz 10 ein- und durch den anderen Führungskanal 17 wieder aus dem Schlitz 10 austritt, so daß der Faden 19 im Ankerimplantat 2 eine U-förmige Schleife ausbildet.

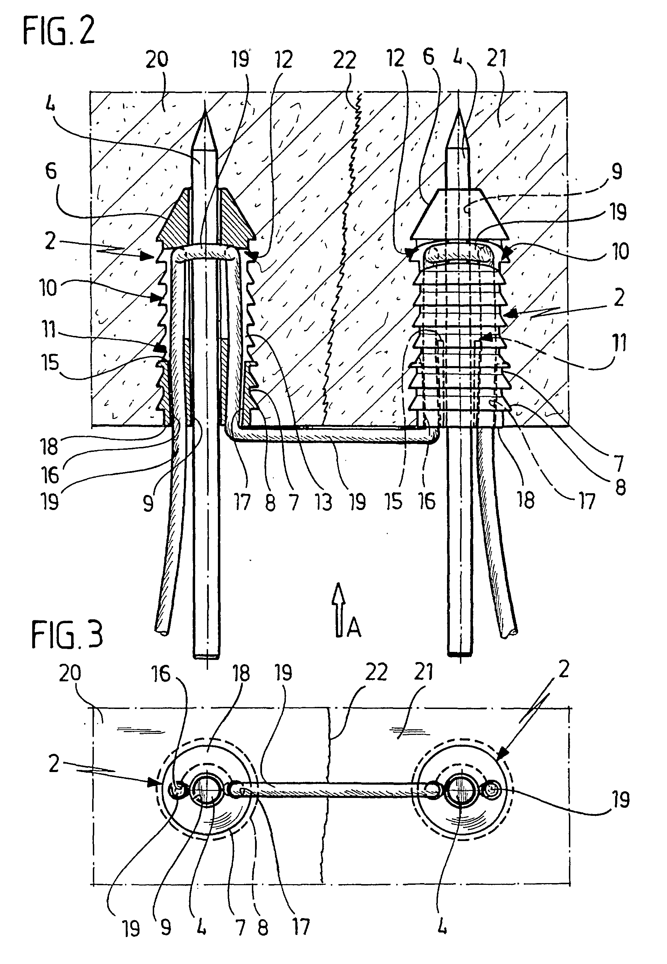

Wenn in den Längskanal 9 des Ankerimplantates 2 der Führungsdraht 4 eingeschoben ist, wie dies beispielsweise in Figur 1 dargestellt ist, dann kann der Faden 19 nicht in den Klemmabschnitt 11 des Schlitzes 10 eintreten, da dieser von dem Führungsdraht 4 ausgefüllt ist, der Faden 19 muß dann in den Verschiebeabschnitt 12 ausweichen, wobei er sich halbkreisförmig an die Außenseite des Führungsdrahtes 4 anlegt (Figuren 1 und 2).

Der Abstand der Seitenwände 13 und 14 des Schlitzes 10 ist im Verschiebeabschnitt 12 größer als der Durchmesser des Fadens 19, so daß der Faden 19 im Verschiebeabschnitt frei verschiebbar ist.

Wenn der Führungsdraht 4 aus dem Längskanal 9 entfernt ist, kann der Faden 19 in den Klemmabschnitt 11 gleiten, dies erfolgt insbesondere dann, wenn der Faden 19 an den beiden aus dem Ankerimplantat 2 herausführenden Fadenenden gespannt wird. Dadurch gelangt der Faden 19 zwischen die zum Boden 15 hin konvergierenden Seitenwände 13 und 14 und wird dort eingeklemmt, so daß eine Längsverschiebung des Fadens nicht mehr möglich ist.

Zum Anlegen der beschriebenen Ankerimplantate 2 werden zwei derartige Ankerimplantate in zwei miteinander zu verbindende Gewebeteile 20, 21 eingesteckt. Dazu wird an jedem Gewebeteil 20 bzw. 21 ein Instrument 1 angesetzt, d.h. es wird ein Führungsdraht 4 eingeschoben, und längs des Führungsdrahtes 4 wird ein Ankerimplantat 2 in das Gewebeteil 20, 21 vorgeschoben. Der in den beiden Ankerimplantaten 2 verlaufende Faden 19 ist dabei beiden Ankerimplantaten 2 gemeinsam, d.h. dieser Faden 19 überbrückt die Verbindungsfläche 22 der beiden Gewebeteile 20, 21.

Solange die Führungsdrähte 4 noch in den Gewebeteilen 20 und 21 verbleiben, ist der gemeinsame Faden 19 in beiden Ankerimplantaten 2 in Längsrichtung frei verschiebbar.

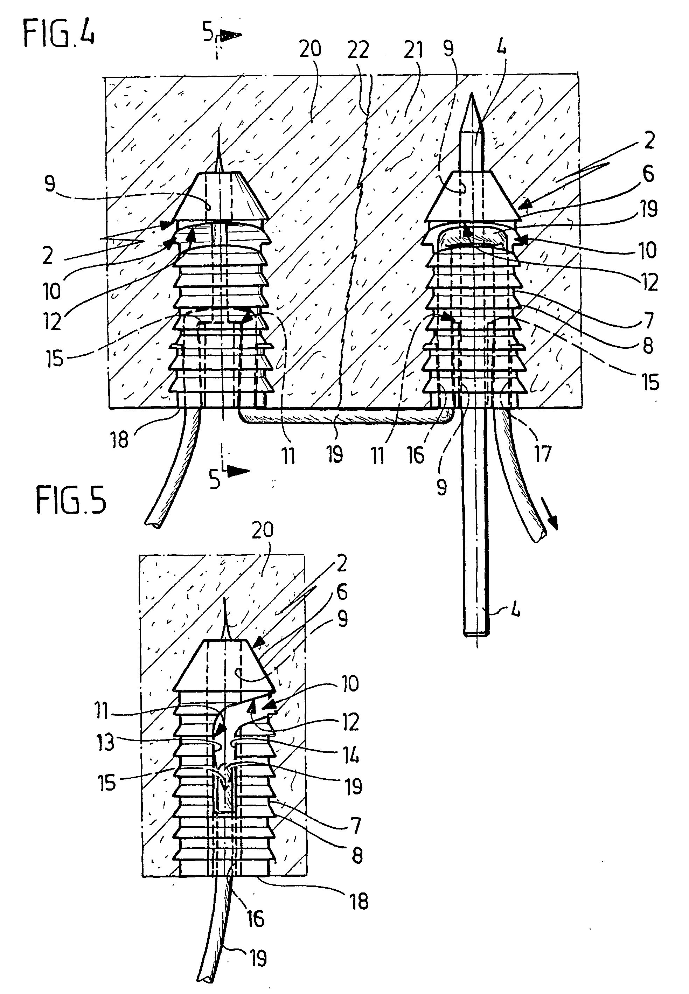

Durch Herausziehen eines Führungsdrahtes 4 und durch Spannen des Fadens 19 wird der Faden 19 in dem Ankerimplantat 2, aus dem der Führungsdraht 4 herausgezogen ist, in den Klemmabschnitt 11 verschoben und dadurch im Inneren des Ankerimplantates 2 festgelegt (Figur 4, linkes Ankerimplantat). Es ist jetzt möglich, den Faden 19 in dem anderen Ankerimplantat 2 so zu verschieben, bis die gewünschte Fadenspannung erreicht ist, durch die die beiden Gewebeteile 20 und 21 zusammengezogen werden. Sobald dies der Fall ist, kann auch bei dem zweiten Ankerimplantat der Führungsdraht 4 herausgezogen werden, so daß dann der Faden 19 auch in dem zweiten Ankerimplantat 2 in den Klemmabschnitt 11 gleitet und dort verklemmt wird (Figur 6). Ohne Knotenbildung sind damit die beiden Ankerimplantate 2 mit der gewünschten Fadenlänge und damit auch mit der zum Zusammenfügen der Gewebeteile 20 und 21 gewünschten Spannung miteinander verbunden.

Auf demselben Faden 19 können auch noch weitere Ankerimplantate 2 in derselben Weise aufgereiht werden, so daß mit demselben Faden eine größere Anzahl von Ankerimplantaten 2 in der beschriebenen Weise miteinander verspannt werden können. Nach dem Setzen des ersten Ankerimplantates 2 läßt sich dies einfach dadurch erreichen, daß sukzessive weitere Ankerimplantate 2 gesetzt werden, daß bei den gesetzten Ankerimplantaten 2 der Faden gespannt wird, daß dann das Führungsinstrument mit dem Führungsdraht 4 abgezogen wird und daß dadurch der Faden auch bei dem neu eingesetzten Ankerimplantat 2 festgelegt wird. Dies ist ein sehr einfacher Vorgang, der schnell vorgenommen werden kann, insbesondere entfallen die Zeiten zur Herstellung eines Knotens, außerdem wird dadurch kompliziertes Handhaben der Fadenenden überflüssig, welches insbesondere bei endoskopischer Operationsweise große Schwierigkeiten bereiten kann.

## Patentansprüche

1. Faden-Anker-System zum Verbinden von Gewebeteilen (20, 21), insbesondere zur Versorgung von Meniskusrissen, mit mindestens zwei pfeil- oder stiftförmigen Ankerimplantaten (2), die über einen flexiblen, an den Ankerimplantaten (2) festgelegten Faden miteinander verbunden sind, **dadurch gekennzeichnet, daß** mindestens eines der Ankerimplantate (2) einen durchgehenden Längskanal (9) zur Aufnahme eines längsverschieblichen Kernes (4) aufweist, daß an diesem Ankerimplantat (2) eine Fadenführung (10) zur längsverschieblichen Aufnahme eines allen Ankerimplantaten (2) gemeinsamen Fadens (19) angeordnet ist, daß der Fadenführung (10) eine Klemmeinrichtung (13, 14) zur Festlegung des Fadens (19) in der Fadenführung (10) zugeordnet ist und daß die Klemmeinrichtung (13, 14) unwirksam ist, solange sich der Kern (4) in dem Längskanal (9) befindet, und erst wirksam wird, wenn der Kern (4) aus dem Längskanal (9) herausgezogen wird.

2. Faden-Anker-System nach Anspruch 1, **dadurch gekennzeichnet, daß** ein erstes Ankerimplantat (2) fest mit dem Faden (19) verbunden ist und alle weiteren Ankerimplantate (2) einen Längskanal (9) mit herausziehbarem Kern (4) und eine Fadenführung (10) mit Klemmeinrichtung (13, 14) aufweisen.

3. Faden-Anker-System nach Anspruch 2, **dadurch gekennzeichnet, daß** auch das erste Ankerimplantat (2) einen durchgehenden Längskanal (9) aufweist.

4. Faden-Anker-System nach Anspruch 1, **dadurch gekennzeichnet, daß** alle Ankerimplantate (2) einen Längskanal (9) mit herausziehbarem Kern (4) und eine Fadenführung (10) mit Klemmeinrichtung (13, 14) aufweisen.

5. Faden-Anker-System nach einem der voranstehenden Ansprüche, **dadurch gekennzeichnet, daß** der Kern (4) ein Führungsdraht ist.

6. Faden-Anker-System nach einem der voranstehenden Ansprüche, **dadurch gekennzeichnet, daß** die Ankerimplantate (2) an ihrer Mantelfläche (7) Widerhaken (8) tragen.

7. Faden-Anker-System nach einem der voranstehenden Ansprüche, **dadurch gekennzeichnet, daß** das Ankerimplantat (2) und/oder der Faden (19) aus resorbierbarem Kunststoffmaterial bestehen.

8. Faden-Anker-System nach einem der voranstehenden Ansprüche, **dadurch gekennzeichnet, daß** die Fadenführung (10) einen den Längskanal (9) durchsetzenden Klemmabschnitt (11) und einen neben dem Längskanal (9) angeordneten Verschiebeabschnitt (12) aufweist, daß der in das Ankerimplantat (2) eingeschobene Kern (4) den Klemmabschnitt (11) gegenüber dem Verschiebeabschnitt (12) absperrt und daß die Klemmeinrichtung (13, 14) in dem Klemmabschnitt (11) angeordnet ist.

9. Faden-Anker-System nach Anspruch 8, **dadurch gekennzeichnet, daß** die Klemmeinrichtung (13, 14) ein sich zu seinem Boden (15) hin verengender Schlitz (10) ist, in den der Faden (19) eingelegt ist und zwischen dessen Seitenwänden (13, 14) er eingeklemmt wird, wenn er gegen den Boden (15) des Schlitzes (10) verschoben wird.

10. Faden-Anker-System nach Anspruch 9, **dadurch gekennzeichnet, daß** sich an den Schlitz (10) beidseitig zum Boden (15) des Schlitzes (10) hin abgewinkelte Führungen (16, 17) für den Faden (19) anschließen.

11. Faden-Anker-System nach Anspruch 10, **dadurch gekennzeichnet, daß** die Führungen (16, 17) als seitlich offene oder geschlossene Kanäle ausgebildet sind, die zu beiden Seiten des Längskanals (9) im wesentlichen parallel zu diesem im Ankerimplantat (2) verlaufen und an einem Ende in den Boden (15) des Schlitzes (10) einmünden.

12. Faden-Anker-System nach einem der Ansprüche 9 bis 11, **dadurch gekennzeichnet, daß** der Schlitz (10) das Ankerimplantat (2) diametral durchsetzt.

13. Faden-Anker-System nach einem der Ansprüche 9 bis 12, **dadurch gekennzeichnet, daß** die Seitenwände (13, 14) des Schlitzes (10) zum Boden (15) hin spitzwinkelig zusammenlaufen.

14. Faden-Anker-System nach Anspruch 13, **dadurch gekennzeichnet, daß** die Seitenwände (13, 14) im Querschnitt konkav gebogen sind.

15. Faden-Anker-System nach einem der Ansprüche 9 bis 14, **dadurch gekennzeichnet, daß** der Schlitz (10) an seinem dem Boden (15) gegenüberliegenden Ende in den außerhalb des Längskanals (9) angeordneten, Teil des Schlitze (10) bildenden Verschiebeabschnitt (12) übergeht, wobei die Seitenwände (13, 14) des Schlitzes (10) im Verschiebeabschnitt (12) einen Abstand voneinander haben, der größer ist als der Durchmesser des Fadens (19).

16. Faden-Anker-System nach Anspruch 15, **dadurch gekennzeichnet, daß** die Ebene des Schlitzes (10) im Verschiebeabschnitt (12) gegenüber der Ebene des Schlitzes (10) im Klemmabschnitt (11) abgewinkelt ist.

17. Faden-Anker-System nach Anspruch 16, **dadurch gekennzeichnet, daß** die Abwinkelung zwischen 30° und 90° liegt.

18. Faden-Anker-System nach Anspruch 16 oder 17, **dadurch gekennzeichnet, daß** der Schlitz (10) im Übergangsbereich zwischen Klemmabschnitt (11) und Verschiebeabschnitt (12) gebogen ist.

19. Faden-Anker-System nach einem der Ansprüche 15 bis 18, **dadurch gekennzeichnet, daß** der Schlitz (10) am äußeren Ende des Verschiebeabschnittes (12) an der Umfangsfläche (7) des Ankerimplantats (2) nach außen hin offen ist.

20. Faden-Anker-System nach einem der Ansprüche 4 bis 19, **dadurch gekennzeichnet, daß** der Faden (19) an seinem Ende ein Anschlagelement trägt.

## Claims

1. A thread-and-anchor system for connecting tissue parts (20, 21), in particular for the treatment of meniscal tears, comprising at least two arrow- or pin-shaped anchor implants (2) which are connected to one another by a flexible thread secured to the anchor implants (2), **characterised in that** at least one of the anchor implants (2) has a continuous longitudinal channel (9) for receiving a longitudinally displaceable core (4), **in that** a thread guide (10) is arranged on this anchor implant (2) and is provided for longitudinally displaceably receiving a thread (19) which is common to all the anchor implants (2), **in that** a clamping means (13, 14) for securing the thread (19) in the thread guide (10) is associated with the thread guide (10), and **in that** the clamping means (13, 14) is ineffective as long as the core (4) is located in the longitudinal channel (9) and only becomes effective when the core (4) is withdrawn from the longitudinal channel (9).

2. A thread-and-anchor system according to claim 1, **characterised in that** a first anchor implant (2) is fixedly connected to the thread (19), and all further anchor implants (2) have a longitudinal channel (9) with a removable core (4) and a thread guide (10) with a clamping means (13, 14).

3. A thread-and-anchor system according to claim 2, **characterised in that** the first anchor implant (2) also has a continuous longitudinal channel (9).

4. A thread-and-anchor system according to claim 1, **characterised in that** all the anchor implants (2) have a longitudinal channel (9) with a removable core (4) and a thread guide (10) with a clamping means (13, 14).

5. A thread-and-anchor system according to any one of the preceding claims, **characterised in that** the core (4) is a guide wire.

6. A thread-and-anchor system according to any one of the preceding claims, **characterised in that** the anchor implants (2) carry barbs (8) on their outer surface (7).

7. A thread-and-anchor system according to any one of the preceding claims, **characterised in that** the anchor implant (2) and/or the thread (19) comprise resorbable plastics material.

8. A thread-and-anchor system according to any one of the preceding claims, **characterised in that** the thread guide (10) has a clamping portion (11) extending into the longitudinal channel (9) and a displacement portion (12) arranged next to the longitudinal channel (9), **in that** the core (4), which is inserted into the anchor implant (2), isolates the clamping portion (11) from the displacement portion (12), and **in that** the clamping means (13, 14) is arranged in the clamping portion (11).

9. A thread-and-anchor system according to claim 8, **characterised in that** the clamping means (13, 14) is a slot (10) which narrows towards its base (15) and into which the thread (19) is placed and between the side walls (13, 14) of which the thread (19) is clamped when it is displaced towards the base (15) of the slot (10).

10. A thread-and-anchor system according to claim 9, **characterised in that** guides (16, 17) for the thread (19) adjoin the slot (10) on both sides and are angled towards the base (15) of the slot (10).

11. A thread-and-anchor system according to claim 10, **characterised in that** the guides (16, 17) are formed as laterally open or closed channels which extend in the anchor implant (2) on both sides of the longitudinal channel (9) so as to be substantially parallel thereto and which lead into the base (15) of the slot (10) at one end.

12. A thread-and-anchor system according to any one of claims 9 to 11, **characterised in that** the slot (10) extends diametrically through the anchor implant (2).

13. A thread-and-anchor system according to any one of claims 9 to 12, **characterised in that** the side walls (13, 14) of the slot (10) converge at an acute angle towards the base (15).

14. A thread-and-anchor system according to claim 13, **characterised in that** the side walls (13, 14) are concave in cross-section.

15. A thread-and-anchor system according to any one of claims 9 to 14, **characterised in that** the slot (10) merges at its end opposite the base (15) into the displacement portion (12) arranged outside the longitudinal channel (9) and forming part of the slot (10), the side walls (13, 14) of the slot (10) in the displacement portion (12) having mutual spacing which is greater than the diameter of the thread (19).

16. A thread-and-anchor system according to claim 15, **characterised in that** the plane of the slot (10) in the displacement portion (12) is at an angle to the plane of the slot (10) in the clamping portion (11).

17. A thread-and-anchor system according to claim 16, **characterised in that** the angle lies between 30° and 90°.

18. A thread-and-anchor system according to claim 16 or 17, **characterised in that** the slot (10) is curved in the transition region between the clamping portion (11) and the displacement portion (12).

19. A thread-and-anchor system according to any one of claims 15 to 18, **characterised in that** the slot (10) is open towards the outside at the outer end of the displacement portion (12) on the circumferential surface (7) of the anchor implant (2).

20. A thread-and-anchor system according to any one of claims 4 to 19, **characterised in that** the thread (19) carries a stop member at its end.

## Revendications

1. Système d'ancrage de fil pour relier des parties de tissu (20, 21), en particulier pour soigner des ruptures de ménisques, comportant au moins deux implants d'ancrage (2) en forme de flèche ou de tige, qui sont reliés l'un à l'autre via un fil flexible immobilisé sur les implants d'ancrage (2), **caractérisé en ce qu'**au moins un des implants d'ancrage (2) présente un canal longitudinal (9) d'un seul tenant destiné à recevoir un noyau (4) à déplacement longitudinal, **en ce que** sur cet implant d'ancrage (2) est agencé un guide-fil (10) destiné à recevoir à déplacement longitudinal un fil (19) commun à tous les implants d'ancrage (2), **en ce qu'**au guide-fil (10) est associé un dispositif de serrage (13, 14) pour immobiliser le fil (19) dans le guide-fil (10), **en ce que** le dispositif de serrage (13, 14) est inactif tant que le noyau (4) se trouve dans le canal longitudinal (9) et ne devient actif que lorsque le noyau (4) est retiré hors du canal longitudinal (9).

2. Système d'ancrage de fil selon la revendication 1, **caractérisé en ce qu'**un premier implant d'ancrage (2) est relié de manière solidaire au fil (19) et tous les autres implants d'ancrage (2) présentent un canal longitudinal (9) avec noyau (4) extractible et un guide-fil (10) avec dispositif de serrage (13, 14).

3. Système d'ancrage de fil selon la revendication 2, **caractérisé en ce que** le premier implant d'ancrage (2) aussi présente un canal longitudinal (9) traversant.

4. Système d'ancrage de fil selon la revendication 1, **caractérisé en ce que** tous les implants d'ancrage (2) présentent un canal longitudinal (9) avec noyau extractible (4) et un guide-fil (10) avec dispositif de serrage (13).

5. Système d'ancrage de fil selon l'une des revendications précédentes, **caractérisé en ce que** le noyau (4) est un fil métallique de guidage.

6. Système d'ancrage de fil selon l'une des revendications précédentes, **caractérisé en ce que** les implants d'ancrage (2) portent des ardillons sur leur surface enveloppe (7).

7. Système d'ancrage de fil selon l'une des revendications précédentes, **caractérisé en ce que** l'implant d'ancrage (2) et/ou le fil (19) est en matière plastique résorbable.

8. Système d'ancrage de fil selon l'une des revendications précédentes, **caractérisé en ce que** le guide-fil (10) présente un tronçon de serrage (11) traversant le canal longitudinal (9) et un tronçon de déplacement (12) agencé à côté du canal longitudinal (9), **en ce que** le noyau (4) introduit dans l'implant d'ancrage (2) bloque le tronçon de serrage (11) par rapport au tronçon de déplacement (12), et **en ce que** le dispositif de serrage (13, 14) est agencé dans le tronçon de serrage (11).

9. Système d'ancrage de fil selon la revendication 8, **caractérisé en ce que** le dispositif de serrage (13, 14) est une fente (10) se rétrécissant vers son fond (15), dans laquelle le fil (19) est placé et entre les parois latérales (13, 14) de laquelle il est serré lorsqu'il est déplacé contre le fond (5) de la fente (10).

10. Système d'ancrage de fil selon la revendication 9, **caractérisé en ce qu'**à la fente (10) se raccordent des guidages (16, 17) pour le fil, coudés des deux côtés vers le fond (15) de la fente (10).

11. Système d'ancrage de fil selon la revendication 10, **caractérisé en ce que** les guidages (16, 17) sont réalisés sous forme de canaux ouverts ou fermés latéralement, qui s'étendent dans l'implant d'ancrage (2) des deux côtés du canal longitudinal (9) sensiblement parallèlement à celui-ci, et débouchent à une extrémité dans le fond (15) de la fente (10).

12. Système d'ancrage de fil selon l'une des revendications 9 à 11, **caractérisé en ce que** la fente (10) traverse diamétralement l'implant d'ancrage (2).

13. Système d'ancrage de fil selon l'une des revendications 9 à 12, **caractérisé en ce que** les parois latérales (13, 14) de la fente (10) convergent en angle aigu vers le fond (15).

14. Système d'ancrage de fil selon la revendication 13, **caractérisé en ce que** les parois latérales (13, 14) sont incurvées de manière concave en section transversale.

15. Système d'ancrage de fil selon l'une des revendications 9 à 14, **caractérisé en ce que** la fente (10) se transforme à son extrémité opposée au fond (15) dans le tronçon de déplacement (12) faisant partie de la fente (10) et agencé en dehors du canal longitudinal (9), les parois latérales (13, 14) de la fente (10) ayant dans le tronçon de déplacement (12) une distance mutuelle qui est supérieure au diamètre du fil (19).

16. Système d'ancrage de fil selon la revendication 15, **caractérisé en ce que** le plan de la fente (10) dans le tronçon de déplacement (12) est coudé par rapport au plan de la fente (10) dans le tronçon de serrage (11).

17. Système d'ancrage de fil selon la revendication 16, **caractérisé en ce que** le coudage est entre 30° et 90°.

18. Système d'ancrage de fil selon l'une ou l'autre des revendications 16 et 17, **caractérisé en ce que** la fente (10) est incurvée dans la région de transition entre le tronçon de serrage (11) et le tronçon de déplacement (12).

19. Système d'ancrage de fil selon l'une des revendications 15 à 18, **caractérisé en ce que** la fente (10) est ouverte vers l'extérieur à l'extrémité extérieure du tronçon de déplacement (12) sur la surface périphérique (7) de l'implant d'ancrage (2).

20. Système d'ancrage de fil selon l'une des revendications 4 à 19, **caractérisé en ce que** le fil (19) porte à son extrémité un élément de butée.
